# EUROPEAN PATENT SPECIFICATION

(11) **EP 3 138 601 B1**
(45) Date of publication and mention of the grant of the patent: **02.01.2019**
(21) Application number: 15194784.3
(22) Date of filing: 16.11.2015
(51) Int. Cl.: A61M 15/00

(54) **DRY POWDER INHALER**
TROCKENPULVERINHALATOR
INHALATEUR DE POUDRE SÈCHE

(30) Priority: 04.09.2015 EP 15183956
(43) Date of publication of application: 08.03.2017
(73) Proprietor: Esteve, Victor, 13.306-530 Itu (BR)
(72) Inventor: Esteve, Victor, 13.306-530 Itu (BR)
(74) Representative: DREISS Patentanwälte PartG mbB

(56) References cited:
- EP-A2- 1 270 034
- WO-A1-94/06498
- WO-A1-2015/006838
- US-A- 3 518 992
- US-A- 3 837 341
- US-A- 5 797 391
- US-A1- 2015 114 391
- US-A1- 2015 231 344

## Description

The invention relates to a dry powder inhaler for a capsule containing dry powder, the inhaler comprising a housing having a capsule receptacle, two actuator buttons arranged on opposing sides of the housing and two perforation needles, each needle being fixedly connected to an actuator button and movable relative to the housing towards each other from a normal position to a perforation position along an actuation direction to perforate a capsule arranged in the capsule receptacle.

Such a dry powder inhaler is known, for example from EP 1 270 034 A1 or from US 2003/0000523. Those dry powder inhalers are typically non-pressurized inhalers. A capsule which is arranged in the capsule receptacle of the dry powder inhaler can be pierced by the needles. When air is sucked in by a patient through a mouthpiece, the capsule is lifted from the capsule receptacle into a capsule chamber and rotates in the capsule chamber. Upon rotation of the capsule, dry powder contained in the capsule is dispersed by centrifugal forces during rotation and mixed with air such that it can be inhaled by a patient.

In order to disperse the dry powder contained in the capsule, the capsule has to be perforated by the needles. Nevertheless, the perforation technique in the prior art inhalers is either complicated to manufacture or needs a lot of moving parts which makes the inhalers difficult to assemble and more expensive.

It is furthermore a problem of the known dry powder inhalers that the dosage of dry powder is limited. When piercing the capsule directly in its hemispherical ends, the amount of powder to be dispersed during inhalation is limited because the flow of the dry powder out of the pierced openings is obstructed during rotational movement of the capsule due to the arrangement of the openings. The obstruction can be caused by cut-out portions of the capsule shell which are cut out by the needles when piercing the capsule. Moreover, the known dry powder inhalers are limited in the maximum dosage of dry powder. In certain areas of application a high dosage in the range of about 25 to about 200mg of dry powder is required such as in treatments or clinical tests for cystic fibrosis, tuberculosis, pulmonary artery hypertension, neurologic and congenital disorders, Parkinson, asthma, Chronic obstructive pulmonary disease (COPD) etc.

Moreover, known inhalers often have the problem that a big variation in the inhalation resistance can occur depending on the pulmonary capacity of a user or patient, wherein a high inhalation resistance at a typical pulmonary capacity of about 60 l/min should be avoided. US 3 837 341 A, US 3 518 992 A, WO 94/06498 A1, US 2015/231344 A1 and WO 2015/006838 A1 show inhalers with features of the preamble of claim 1. US 5 797 391 A discloses a spring with limbs, each limb comprising a capsule-piercing pin...

It is therefore an object of the present invention to provide a cost effective dry powder inhaler for dispersion of dry powder which provides for easy handling of large dosages of dry powder without a high inhalation resistance.

This object is solved by a dry powder inhaler according to the features of claim 1. Such a dry powder inhaler is characterized in that a first end of each actuator button is connected to the housing at a lower portion of the housing and in that a second free end of each actuator button is movable into the perforation position such that the perforation needle is moved along a circular line. Preferably, the housing is made of two parts, wherein the housing comprises a base body with a capsule chamber and a covering body connected to the base body. With this dry powder inhaler, a cheap and simple construction can be provided which makes the perforation of a capsule arranged in the capsule receptacle easy to be achieved. By arranging two actuator buttons on opposing sides of the housing and connecting each actuator button to the housing at a lower portion of the housing, wherein a perforation needle is arranged at a second free end of the actuator button, the actuator buttons can be pre-loaded into the normal position by use of only one spring element arranged between the actuator buttons. Therefore, a simple and cheap construction can be achieved. The capsule receptacle can be arranged in an angle with respect to the actuation direction. Nevertheless, it is also possible that the capsule receptacle is arranged parallel to the actuation direction.

According to a first advantageous embodiment of the inhaler, the first end of each actuator button is pivotably attached to the housing.

According to the invention, a first end of each actuator button is connected to the housing via a ball joint snap-on connection pivoting around a pivot axis. By providing a ball joint snap-on connection, the actuator buttons can be attached to the lower portion of the housing by engaging the ball point snap-on connection. Advantageously, the actuator buttons are pivotable around a pivoting angle of about 5 to 15°, preferably of about 10°. In another preferred embodiment of the inhaler, the ball joint snap-on connection comprises hemi-spherical protrusions arranged on the housing and hemi-spherical recesses arranged on the actuator buttons complementary to the hemi-spherical protrusions, wherein the protrusions and recesses extend along the pivot axis. In order to engage the ball joint snap-on connection, the hemi-spherical protrusions are preferably arranged on the housing such that they can be elastically pushed into a release position along the direction of the pivot axis. In the release position, the recesses of the actuator buttons and the protrusions of the housing can be superimposed such that the protrusions can engage the recesses and elastically move back into an engagement position of the ball joint snap-on connection.

It is particularly preferred if the perforation needles are arranged in an area of the second free end of each actuator button spaced away from the first end of the actuator button. Preferably, the perforation needles are arranged on the inside surfaces of the actuator buttons facing each other. By arranging the perforation needles in an area of the second free end of each actuator button, a large movement range can be provided despite connecting a first end of each actuator button to the housing.

In a particularly preferred embodiment, the dry powder inhaler comprises a spring element acting upon the actuator buttons such that the actuator buttons are pre-loaded into the normal position. Preferably, the spring element is a helical spring.

In another preferred embodiment of the dry powder inhaler, the housing comprises two openings configured to receive the actuator buttons such that an outside surface of the housing and the actuator buttons is substantially flush when the actuator buttons are in the normal position. It is particularly preferred if the actuator buttons close the openings in the housing in the normal position in a substantially airtight manner such that no or substantially no air can pass between the actuator buttons and the opening edges.

Further details and advantages of the invention can be taken from the following description, on the basis of which the embodiments of the invention that are represented in the Figures are described and explained in more detail.

Respective figures are showing:
- Figure 1: a side view of a dry powder inhaler according to the invention;
- Figure 2: a perspective view of the dry powder inhaler of Figure 1;
- Figure 3: a sectional view of a part of the dry powder inhaler of Figures 1 and 2
- Figure 4: a perspective view showing parts of the dry powder inhaler of Figures 1 to 3;
- Figure 5: a perspective view of a base body of the dry powder inhaler of Figures 1 to 4;
- Figure 6: a perspective top view of the base body of Figure 5;
- Figure 7: a top perspective view of a dry powder inhaler of Figures 1 to 4 in an open position;
- Figure 8: a top view of the base body of Figures 5 and 6;
- Figure 9: a capsule and two needles in a piercing position;
- Figure 10: a side view of a needle of the dry powder inhaler of Figures 1 to 4:
- Figure 11: a top view of the needle of Figure 10;
- Figure 12: a front view of the needle of Figures 10 and 11; and;
- Figure 13: a schematic view of a pierced capsule during inhalation.

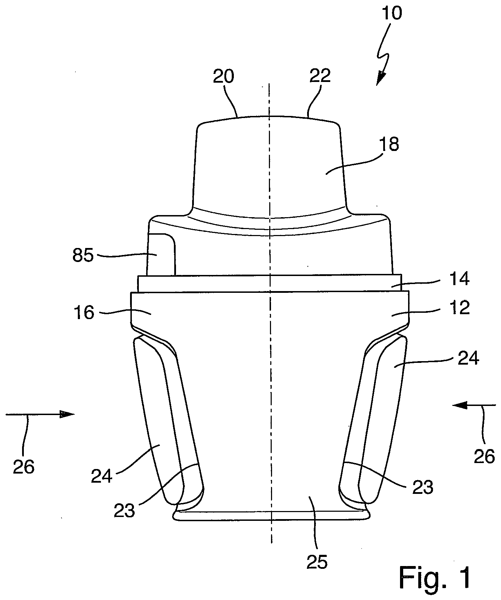

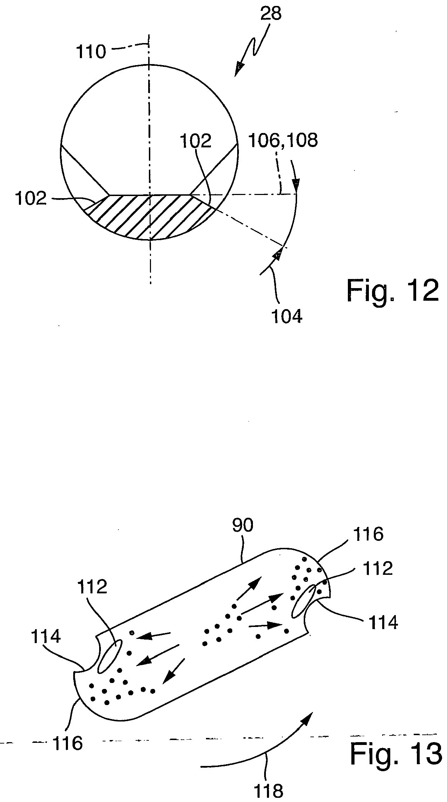

Figures 1 to 12 show a dry powder inhaler 10 for a capsule containing dry powder (shown in Figure 13). The dry powder inhaler 10 comprises a housing 12 having a base body 14 and a covering body 16. The dry powder inhaler 10 furthermore comprises a mouthpiece 18. The mouthpiece 18 has a mouth portion 20 having a central opening 22 for inhaling air that mixes with dry powder contained in a capsule arranged in the dry powder inhaler 10.

The dry powder inhaler 10 comprises two actuator buttons 24 arranged on opposing sides of the housing 12 which are movable relative to the housing 12 towards each other along an actuation direction which is indicated by arrows 26 from a normal position to a perforation position to perforate a capsule arranged in the dry powder inhaler 10. The housing 12 comprises two openings 23 configured to receive the actuator buttons 24 such than an outside surface 25 of the housing 12 and the actuator buttons 24 is substantially flush when the actuator buttons 24 are in the normal position.

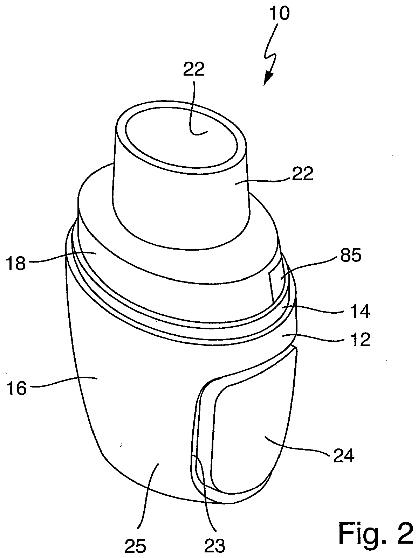

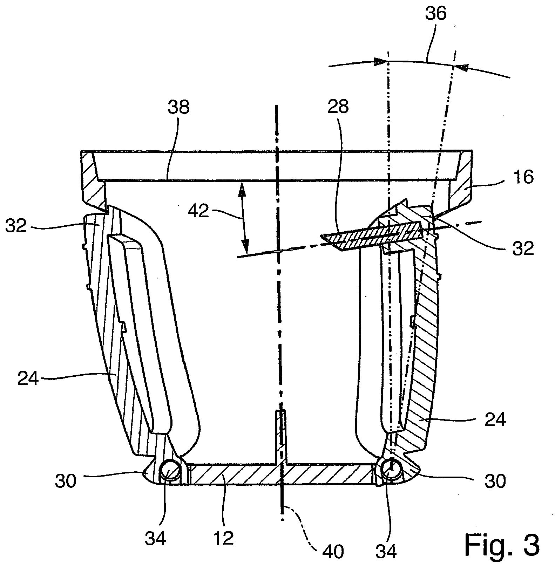

Figure 3 shows a sectional view of a part of the dry powder inhaler 10 of Figures 1 and 2. Each actuator button 24 is attached the covering body 16, i.e. the housing 12, wherein a perforation needle 28 is provided being fixedly connected to an actuator button 24. Figure 3 shows only one perforation needle 28 because the needles 28 are arranged with an offset concerning the cutting plane of Figure 3.

A first end 30 of each actuator button 24 is connected to the housing 12, i.e. the covering body 16 in a lower portion of the housing 12. The perforation needles 28 are arranged in an area of a second free end 32 of the actuator buttons 24. This second free end 32 is spaced away from the first end 30. The second free end 32 of the actuator buttons 24 is movable from the normal position into the perforation position shown in Figure 3 such that a tip of the perforation needle 28 is moved along a circular line.

The first end 30 of each actuator button 24 is pivotably attached to the housing 12 by use of a ball joint snap-on connection 34 pivoting around a pivot axis, wherein the actuator button is pivotable around a pivoting angle 36 of about 5° to 15°, preferably of about 10°. Accordingly, the perforation needles 28 are inclined with respect to a plane 38 which is perpendicular to a longitudinal axis 40 of the dry powder inhaler 10 in a perforation angle 42 of about 5° to 15°, preferably of about 10° when the actuator buttons 24 are in the perforation position.

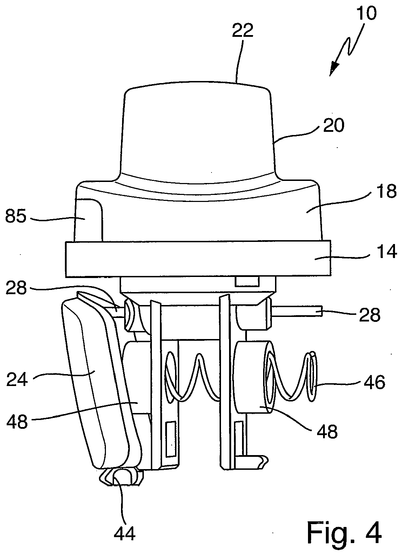

The ball joint snap-on connection 34 comprises hemi-spherical protrusions (not shown in the drawings) arranged on the housing 12 and hemi-spherical recesses 44 (shown in Figure 4) arranged on the actuator buttons 24, wherein the protrusions and recesses extend along the pivot axis and wherein the protrusions engage the recesses.

By arranging the two actuator buttons 24 on opposing sides of the housing 12 and connecting each actuator button 24 to the housing 12 at a lower portion of the housing 12, the actuator buttons 24 can be pre-loaded into the normal position by use of only one spring element.

Such a spring element is shown in Figure 4 which shows a perspective view with of parts of the dry powder inhaler. The dry powder inhaler 10 comprises a helical spring 46 arranged in shell-like spring-guiding members 48 of the base body 12 and acting upon the actuator buttons 24 such that the actuator buttons 24 are pre-loaded into the normal position against the direction depicted by arrows 26.

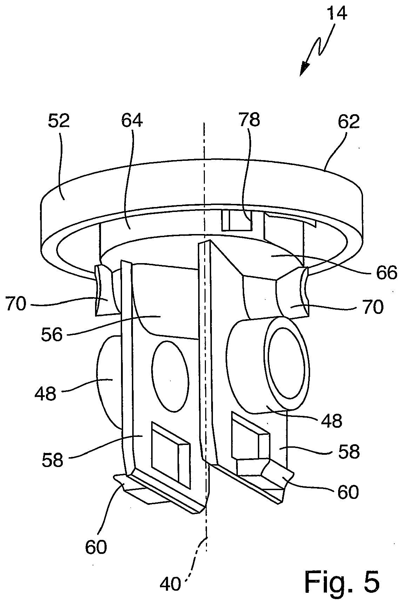

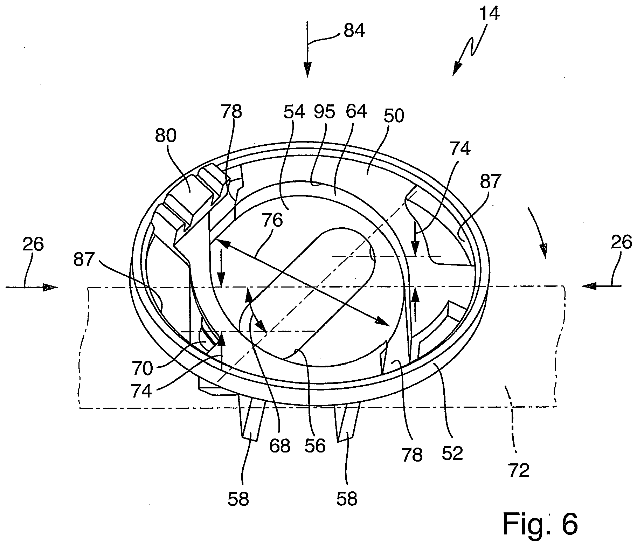

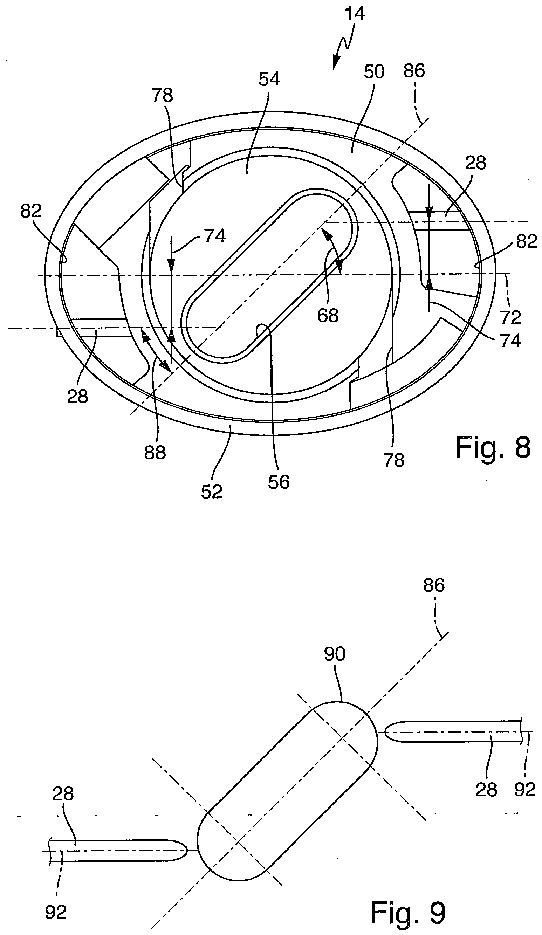

The base body 14 of the dry powder inhaler 10 which is made of one piece is shown in more detail in Figures 5 and 6. Figure 8 furthermore shows a top view of the base body 14 of Figures 5 and 6. The base body 14 has an elliptical or oval base body plate 50 and an oval cylindrical shoulder 52 extending perpendicular to the base body plate 50 and connected to the base body plate 50. The base body 14 has a capsule chamber 54 having a capsule receptacle 56 for receiving a dry powder capsule and two downwardly extending plate members 58 that are substantially parallel to each other. The spring-guiding members 48 are arranged coaxially to the actuation direction 26 wherein the plate members 58 are arranged perpendicular to the actuation direction 26. At a free end of the plate members 58, bearing sections 60 for the actuator buttons 24 are provided.

The capsule chamber 54 is open towards an upper end portion 62 of the base body 14. The capsule chamber 54 has a circular cylindrical section 64 and a hemi-spherical or conical end section 66, wherein the capsule receptacle 56 is arranged in the hemi-spherical end section 66 of the capsule chamber 54. The capsule chamber 54 has a volume of about 2500 to about 3000 mm³. The capsule receptacle 56 is arranged perpendicular to the longitudinal axis 40 of the dry powder inhaler 10.

The capsule receptacle 56 is furthermore arranged in an inclined angle 68 within the range of 40° to 50° with respect to the actuation direction 26. The base body 14 comprises two hollow needle guiding sections 70 configured to guide the needles 28 and being arranged perpendicular to the longitudinal axis 40 of the inhaler 10 and ending in the capsule receptacle 56. Those needle guiding sections 70 and the needles 28 are arranged parallel to the actuation direction 26 and spaced apart from a middle plane 72 of the base body 14 in a distance 74. The needles 28 are arranged on opposing sides of the middle plane 72.

According to another embodiment not shown in the drawings, the capsule receptacle 56 is not arranged in an inclined angle with respect to the actuation direction 26 but is parallel to the actuation direction 26. In that case the needle guiding sections 70 and the needles 28 are arranged parallel to the actuation direction 26 but are not spaced apart from the middle plane 72 of the base body 14. With this embodiment a capsule arranged in the capsule receptacle 56 can be pierced directly in the hemi-spherical end-section of the capsule.

The circular cylindrical section 64 has a diameter 76 which is bigger than the length of the capsule receptacle 56. The size of the capsule receptacle 56 corresponds with the size of the capsule such that the capsule receptacle 56 is only slightly bigger than the capsule to ensure that the capsule is held in the capsule receptacle 56 when pushing the actuator buttons 24 and piercing the capsule with the needles 28. Preferably, the capsule receptacle 56 is configured to receive a capsule with a volume in the range of about 400 to about 500 mm³.

The base body 14 furthermore has two base body air inlets 78 in the area of the cylindrical shoulder 52 which are ending tangentially into the capsule chamber 54 and are arranged perpendicular to the middle plane 72 of the base body 14. The mouthpiece 18 is fastened to the base body 14 via a hinge. A lower part of the hinge is depicted in Figure 6 and having the reference numeral 80. The base body 14 also comprises two base body ducts 82.

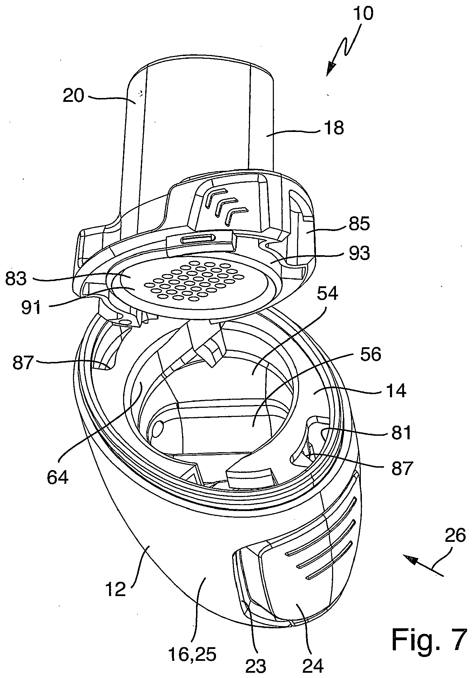

Between the base body 14 and the covering body 16 of the housing 12 an intermediate air chamber 81 is formed. The central opening 22 of the mouthpiece 18 functions as inhaling inlet and is fluidly connected to the capsule chamber 54 via a perforated plate 83 as shown in Figure 7. The mouthpiece 18 has two mouthpiece air inlets 85 on a lateral outside of the mouthpiece which are fluidly connected to the intermediate air chamber 81.

The base body air inlets 78 are also fluidly connected to the intermediate air chamber 81 and to the capsule chamber 54. The base body comprises two base body ducts 87 overlapping with and in fluid connection with the mouthpiece air inlets 85. The base body ducts 87 are also fluidly connected to the intermediate air chamber 81 and end in the intermediate air chamber 81.

On the side facing away from the mouth portion 20, the mouthpiece 18 comprises a cylindrical protrusion 91 configured to extend at least partially into the capsule chamber 54. The perforated plate 83 is arranged in an end-section of the cylindrical protrusion 91. A lateral surface 93 of the cylindrical protrusion 91 is configured to sealingly abut on an inside surface 95 of the capsule chamber 54 such that substantially no air can pass between the inside surface 95 of the capsule chamber 54 and the lateral surface 93 of the cylindrical protrusion.

Figure 8 shows a top view of the base body 14 of Figure 6 when viewing in the direction of arrow 84 in Figure 6. The capsule receptacle 56 has a longitudinal axis 86 which is arranged in an inclined angle 68 within the range of about 40° to about 50° with respect to the actuation direction 26 or the middle plane 72. The needles 28 are piercing a capsule which can be arranged in the capsule receptacle 56 in an angle 88 with respect to the longitudinal axis 86 of the capsule receptacle 56 or the capsule.

Such a capsule 90 is shown in Figure 9. The longitudinal axis 86 of the capsule 90 is arranged to a longitudinal axis 92 of the needles 28 in the angle 88.

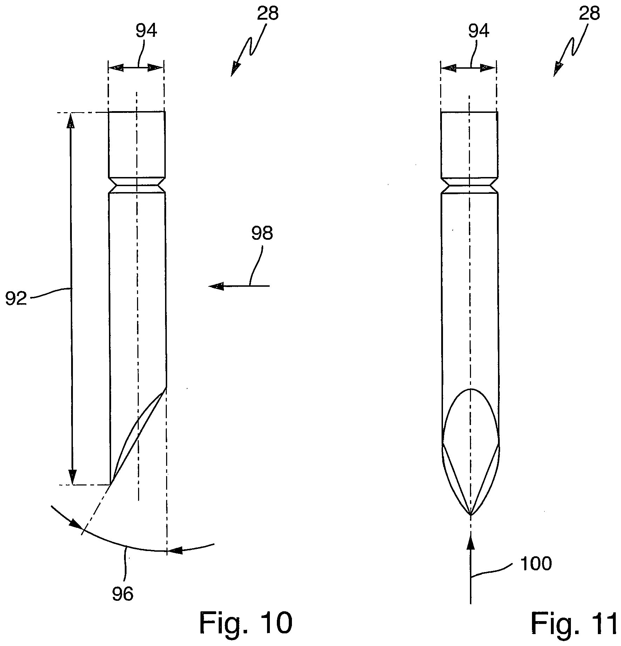

Figure 10 depicts a side view of one of the needles 28 of the dry powder inhaler 10. The needles 28 have a length 92, a needle diameter 94 and a cutting tip angle 96. The needle diameter 94 is in the range of about 1,55 to about 1,85 mm wherein the cutting tip angle 96 is in the range of about 20° to 40°.

Figure 11 shows a top view of the needle 28 of Figure 10 when looking into the direction of arrow 98 in Figure 10.

Figure 12 shows a partial cross-section A-A of the needle 28 of Figures 10 and 11 in a front view when looking into the direction of arrow 100 in Figure 11.

The needles 28 have lateral cutting edges 102 which are arranged in an angle 104 in the range of about 25 to 35° with respect to an axis 106 perpendicular to the longitudinal axis 92 of the needle 28 and lying in a plane 108 parallel to the longitudinal axis 92. The needles 28 are symmetrical to a middle plane 110.

The dry powder inhaler 10 functions as follows:
In order to insert a dry powder capsule 90 into the capsule receptacle 56 of the base body 14, the mouthpiece 18 can be pivoted into an opening position via the hinge 80 as shown in Figure 7. After insertion of the capsule 90, the mouthpiece 18 is pivoted back into a closed position as depicted in Figures 1 and 2. In order to pierce the capsule 90, the actuator buttons 24 are pressed against the force of the helical spring 46 into the actuation direction 26.

The cutting tip angle 96, the lateral cutting edges 102 and the arrangement of the capsule receptacle 56 and the needles 28 provide for an accurate cutting of a shell of the capsule 90 during piercing of the capsule 90 without detaching the cut-out portion from the capsule shell.

Figure 13 depicts a schematic view of a pierced capsule 90 during inhalation. Cut-out portions 112 can be bent inwards into the interior of the capsule 90 in a hinged or flap-like manner.

Because of the comparably large needle diameter 94, comparably big openings 114 can be pierced into the capsule 90 in a transition area of the hemispherical ends 116 and the tubular middle section of the capsule 90.

When a patient or user is using the dry powder inhaler 10, a capsule 90 arranged in the capsule receptacle 56 can be pierced by moving the actuator buttons 24 from the normal position into the perforation position. After piercing the capsule 90, the actuator buttons 24 are moved back into the normal position. When a patient or user is sucking in air through the inhaling inlet 22 of the mouthpiece 18, a partial vacuum in the capsule chamber 54 can be generated wherein air is sucked into the capsule chamber 54 through the at least one base body air inlet 78 generating an air flow that lifts the capsule 90 from the capsule receptacle 56 into the capsule chamber 54 and rotates the capsule 90 in the capsule chamber 54 in the direction of arrow 118 as shown in Figure 13 such that dry powder contained in the capsule 90 can be dispersed.

When sucking in air on the inhaling inlet 22 through the capsule chamber 54, air is directed from the mouthpiece air inlets 85 via the base body ducts 87 and through the intermediate air chamber 81 into the capsule chamber 54 via the base body air inlets 78.

Table 1 and Table 2 shown below show comparative tests of flow resistances of a dry powder inhaler 10 having an intermediate air chamber 81 and flow resistances of a dry powder inhaler without an intermediate air chamber 81.

**Table 1: flow resistance at different air flow**

| inhaler with intermediate air chamber | | |
|---|---|---|
| L/min, | Value | Unit |
| 30 | 0,74 | kPA |
| 60 | 1,56 | kPA |
| 90 | 3,52 | kPA |
| 100 | 3,81 | kPA |

**Table 2: flow resistance at different air flow**

| inhaler without intermediate air chamber | | |
|---|---|---|
| L/min, | Value | Unit |
| 30 | 0,82 | kPA |
| 60 | 2,16 | kPA |
| 90 | 3,83 | kPA |
| 100 | 4,02 | kPA |

By directing the air flow through the intermediate air chamber 81, a dry powder inhaler 10 can be provided which is less stiff at lower flow rates and more stiff at higher flow rates in contrast to a dry powder inhaler which has no intermediate air chamber 81.

Overall, an inhaler 10 with an intermediate air chamber 81 can be provided that has less flow resistance at typical lung capacities. When a user or patient has less lung capacity of about 60L/min, a dry powder inhaler 10 can be provided with significantly less flow resistance in comparison to a dry powder inhaler without an intermediate air chamber 81.

Because of the arrangement of the capsule receptacle 56 and the needles 28, the capsule 90 can be pierced in a position that allows powder to be efficiently dispersed during the rotational movement in an inhalation process while providing little or almost no obstruction to the powder flow.

To sum up, a dry powder inhaler 10 is provided which allows for a high dosage inhalation in the range of about 25 to about 200 mg.

## Claims

1. A dry powder inhaler (10) for a capsule (90) containing dry powder, the inhaler (10) comprising a housing (12) having a capsule receptacle (56), two actuator buttons (24) arranged on opposing sides of the housing (12) and two perforation needles (28), each needle (28) being fixedly connected to an actuator button (24) and movable relative to the housing (12) towards each other from a normal position to a perforation position along an actuation direction to perforate a capsule (90) arranged in the capsule receptacle (56), **characterized in that** a first end (30) of each actuator button (24) is connected to the housing (12) at a lower portion of the housing (12) via a ball joint snap-on connection (34), wherein each actuator button (24) pivots around a respective pivot axis, and **in that** a second free end (32) of each actuator button (24) is movable into the perforation position such that the perforation needle (28) is moved along a circular line.

2. The dry powder inhaler (10) of claim 1, wherein the first end (30) of each actuator button is pivotably attached to the housing (12).

3. The dry powder (10) inhaler of at least one of the foregoing claims, wherein the actuator buttons (24) are pivotable around a pivoting angle (36) of about 5 to 15°, preferably of about 10°.

4. The dry powder inhaler (10) of claim 1 or 3, wherein the ball joint snap-on connection (34) comprises hemi-spherical protrusions arranged on the housing (12) and hemi-spherical recesses arranged on the actuator buttons (24) complementary to the hemi-spherical protrusions, wherein the protrusions and recesses extend along the pivot axis.

5. The dry powder inhaler (10) of at least one of the foregoing claims, wherein the perforation needles (28) are arranged in an area of the second free end (32) of each actuator button (24) spaced away from the first end (30) of the actuator button (24).

6. The dry powder inhaler (10) of at least one of the foregoing claims, wherein the dry powder inhaler (10) comprises a spring element (46) acting upon the actuator buttons (24) such that the actuator buttons (24) are pre-loaded into the normal position.

7. The dry powder inhaler (10) of at least one of the foregoing claims, wherein the housing (12) comprises two openings (23) configured to receive the actuator buttons (24) such that an outside surface (25) of the housing (12) and the actuator buttons (24) is substantially flush when the actuator buttons (24) are in the normal position.

## Patentansprüche

1. Trockenpulverinhalator (10) für eine trockenpulverhaltige Kapsel (90), wobei der Inhalator (10) ein Gehäuse (12) mit einer Kapselaufnahme (56), zwei Betätigungsknöpfen (24), die auf gegenüberliegenden Seiten des Gehäuses (12) angeordnet sind, und zwei Perforationsnadeln (28) umfasst, wobei die Perforationsnadeln (28) fest mit einem Betätigungsknopf (24) verbunden und relativ zum Gehäuse (12) aus einer Normalposition in eine Perforationsposition entlang einer Betätigungsrichtung zueinander beweglich sind, um eine in der Kapselaufnahme (56) angeordnete Kapsel (90) zu perforieren, **dadurch gekennzeichnet, dass** ein erstes Ende (30) jedes Betätigungsknopfes (24) mit dem Gehäuse (12) an einem unteren Abschnitt des Gehäuses (12) über eine Kugelgelenk-Schnappverbindung (34) verbunden ist, wobei jeder Betätigungsknopf (24) um eine jeweilige Schwenkachse schwenkbar ist, und dass ein zweites freies Ende (32) jedes Betätigungsknopfes (24) in die Perforationsposition beweglich ist, so dass die Perforationsnadel (28) entlang einer Kreislinie bewegt wird.

2. Trockenpulverinhalator (10) nach Anspruch 1, wobei das erste Ende (30) jedes Betätigungsknopfes schwenkbar am Gehäuse (12) befestigt ist.

3. Trockenpulverinhalator (10) nach wenigstens einem der vorgenannten Ansprüche, wobei die Betätigungsknöpfe (24) um einen Schwenkwinkel (36) von etwa 5 bis 15°, vorzugsweise von etwa 10°, schwenkbar sind.

4. Trockenpulverinhalator (10) nach Anspruch 1 oder 3, wobei die Kugelgelenk-Schnappverbindung (34) halbkugelförmige Vorsprünge, die am Gehäuse (12) angeordnet sind, und halbkugelförmige Aussparungen, die an den Betätigungsknöpfen (24) angeordnet sind und die zu den halbkugelförmigen Vorsprüngen komplementär sind, umfasst, wobei sich die Vorsprünge und Aussparungen entlang der Drehachse erstrecken.

5. Trockenpulverinhalator (10) nach wenigstens einem der vorgenannten Ansprüche, wobei die Perforationsnadeln (28) in einem Bereich des zweiten freien Endes (32) jeder Betätigungsvorrichtung (24) angeordnet sind, der vom ersten Ende (30) der Betätigungsvorrichtung (24) beabstandet ist.

6. Trockenpulverinhalator (10) nach wenigstens einem der vorstehenden Ansprüche, wobei der Trockenpulverinhalator (10) ein Federelement (46) umfasst, das derart auf die Betätigungsknöpfe (24) wirkt, sodass die Betätigungsknöpfe (24) in der Normalposition vorgespannt sind.

7. Trockenpulverinhalator (10) nach wenigstens einem der vorgenannten Ansprüche, wobei das Gehäuse (12) zwei Öffnungen (23) aufweist, die zur Aufnahme der Betätigungsknöpfe (24) so konfiguriert sind, dass eine Außenfläche (25) des Gehäuses (12) und der Betätigungsknöpfe (24) im Wesentlichen bündig sind, wenn sich die Betätigungsknöpfe (24) in der Normalposition befinden.

## Revendications

1. Une inhalateur de poudre sèche (10) pour une capsule (90) contenant de la poudre sèche, l'inhalateur (10) comprenant un boîtier (12) ayant un réceptacle de capsule (56), deux boutons d'actuation (24) disposés sur des côtés opposés du boîtier (12) et deux aiguilles perforatrices (28), chaque aiguille étant fixement connectée à un bouton d'actuation (24) et mobile par rapport au boîtier (12) et l'une vers l'autre depuis une position normale vers une position de perforation le long d'une direction d'actuation pour perforer une capsule (90) disposée dans un réceptacle de capsule (56), **caractérisé en ce qu'une** première extrémité (30) de chaque bouton d'actuation (24) est connectée au boîtier (12) à une partie inférieure du boîtier (12) via une connexion à bille clipsable (34), dans lequel chaque bouton d'actuation pivote autour d'un axe de pivotement respectif, et **en ce qu'**une seconde extrémité libre (32) de chaque bouton d'actuation (24) est mobile dans un position de perforation de sorte que l'aiguille perforatrice (28) soit déplacée le long d'une ligne circulaire.

2. L'inhalateur de poudre sèche (10) de la revendication 1, dans lequel la première extrémité de chaque bouton d'actuation est attachée au boîtier (12) de façon pivotante.

3. L'inhalateur de poudre sèche (10) d'au moins une des revendications précédentes, dans lequel les boutons d'actuation (24) sont pivotables autour d'un angle de pivotement (36) de 5 à 15°, de préférence d'environ 10°.

4. L'inhalateur de poudre sèche (10) de la revendication 1 ou 3, dans lequel la connexion clipsable à bille (34) comprend des protrusions hémisphériques disposées sur le boîtier (12) et des creux hémisphériques disposés sur les boutons d'actuation (24) complémentaires des protrusions hémisphériques, dans lequel des protrusions et les creux s'étendent sur l'axe de pivotement.

5. L'inhalateur de poudre sèche (10) selon l'un au moins des revendications précédentes, dans lequel les aiguilles perforatrices (28) sont disposées dans une zone de la seconde extrémité libre (32) de chaque bouton d'actuation (24) espacé de la première extrémité (30) du bouton d'actuation (24).

6. L'inhalateur de poudre sèche (10) selon l'un au moins des revendications précédentes, dans lequel l'inhalateur de poudre sèche (10) comprend un élément de ressort (46) agissant sur les boutons d'actuation (24) de sorte que les boutons d'actuation (24) soient précontraints dans la position normale.

7. L'inhalateur de poudre sèche (10) selon l'un au moins des revendications précédentes, dans lequel le boîtier (12) comprend deux ouvertures (23) configurées pour recevoir les boutons d'actuation (24) de sorte qu'une surface externe (25) du boîtier (12) et des boutons d'actuation (24) soit essentiellement de niveau lorsque les boutons d'actuation (24) sont en position normale.
